(19) Europäisches Patentamt
European Patent Office
Office européen des brevets

(11) **EP 3 499 258 A1**

(12) **EUROPÄISCHE PATENTANMELDUNG**

(43) Veröffentlichungstag:
**19.06.2019 Patentblatt 2019/25**

(51) Int Cl.:
*G01R 33/28* (2006.01) *G01R 33/38* (2006.01)

(21) Anmeldenummer: **17206949.4**

(22) Anmeldetag: **13.12.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **BIOTRONIK SE & Co. KG**
**12359 Berlin (DE)**

(72) Erfinder:
• **Ebert, Henning**
**12437 Berlin (DE)**
• **Rump, Jens**
**12049 Berlin (DE)**

(74) Vertreter: **Keck, Hans-Georg**
**Biotronik Corporate Services SE**
**Corporate Intellectual Property**
**Sieversufer 7 - 9**
**12359 Berlin (DE)**

(54) **VORRICHTUNG UND VERFAHREN ZUR BESTIMMUNG EINER LOKALEN EIGENSCHAFT EINES BIOLOGISCHEN GEWEBES MIT EINEM NMR SENSOR**

(57) Die Erfindung betrifft eine Vorrichtung zur Bestimmung einer lokalen Eigenschaft eines biologischen Gewebes (50) mit
- einem flexiblen Schaft (20),
- einer Datenverarbeitungseinrichtung (40) und
- einem NMR-Sensor (30), der an dem distalen Ende des Schafts (20) angeordnet und mit der Datenverarbeitungseinrichtung (40) verbunden ist, wobei der NMR-Sensor (30) ein erstes Sensorelement (34) zur Erzeugung eines statischen Magnetfelds und ein zweites Sensorelement (35) zur Erzeugung eines magnetischen Wechselfelds aufweist,
wobei das distale Ende des Schafts (20) benachbart zu der zu vermessenden Stelle des Gewebes (50) anordenbar ist, wobei die Datenverarbeitungseinrichtung (40) zur Bestimmung der lokalen Eigenschaft des Gewebes (50) an dieser Stelle basierend auf einem zur Datenverarbeitungseinrichtung (40) übertragenen Signal des NMR-Sensors (30) eingerichtet ist. Die Erfindung betrifft ferner einen Katheter mit einer oben angegebenen Vorrichtung sowie ein entsprechendes Verfahren.

FIG. 2

EP 3 499 258 A1

**Beschreibung**

Im Zusammenhang mit der Ablation von biologischem Gewebe, wie beispielsweise zur Optimierung der Reizleitung im Herzen (Elektrophysiologie), Verödung von Nerven (renale Denervation) oder Tumorbehandlung ist die Kenntnis von Gewebeeigenschaften, z. B. der Läsionstiefe bzw. der lokalen Dicke des behandelten Gewebes, für die Beurteilung des therapeutischen Erfolgs des Eingriffs von entscheidender Bedeutung. Die vorliegende Erfindung beschäftigt sich daher mit einer Vorrichtung und einem Verfahren zur Bestimmung einer lokalen Eigenschaft eines biologischen Gewebes sowie mit einem Katheter mit einer solchen Vorrichtung.

Ein bekanntes Verfahren zur nichtmedikamentösen, minimalinvasiven Behandlung von idiopathischen, paroxysmalen, persistierenden oder chronischen Arrhythmien, insbesondere supraventrikularen Arrhythmien, des Herzens ist die intrakardiale Ablation. Hierbei wird bei Vorhofflimmern oder anderen Arrhythmien wie Vorhoffflattern ein Katheter mit einer Elektrode über die venösen Blutgefäße in den rechten Vorhof des Herzens eingeführt und durch die Herzscheidewand hindurch im linken Vorhof platziert. Anschließend werden Muskelgewebs-Areale im linken Vorhof mittels eines durch die Elektrode eingebrachten Hochfrequenzstroms derart verödet (abladiert), dass sogenannte Rotoren (kreisende Erregungen) oder ektopische Aktivierungsquellen beseitigt sowie Pulmonalvenen isoliert werden, welche ursächlich für die Arrhythmien gehalten werden. Alternativ kann analog eine minimalinvasive Behandlung mittels Laser, Kälte, Wärmestrahlung, Mikrowellenenergie oder Partikeltherapie (Teilchentherapie) erfolgen.

Die Erfolgsquote einer Ablation kann gesteigert werden, wenn während der Behandlung die Wirksamkeit der durch die Ablation oder andere minimalinvasive Verfahren induzierten Läsionen zuverlässiger beurteilt werden kann. Dies ist mit den heutigen Verfahren nur eingeschränkt möglich. In vielen Fällen ist deshalb nach einer ersten AF-Ablations-Behandlung (AF = atrial fibrillation) noch eine zweite AF-Ablation erforderlich. Dies führt zu einer erhöhten Belastung des Patienten.

Aus der Druckschrift DE 103 09 245 A1 ist eine Vorrichtung zum Lokalisieren einer Läsion in einem biologischen Gewebeabschnitt bekannt, bei der elektrische Anregungssignale an den Gewebeabschnitt angelegt und elektrische Antwortsignale an mehreren Messorten auf der Oberfläche des Gewebeabschnitts, die sich aufgrund der Anregungssignale dort einstellen, gemessen werden. Aus den Antwortsignalen wird eine Verteilung von elektrischen Dipolmomenten rekonstruiert und die räumliche Lage der Verteilung ausgegeben. Aus diesen Dipolmomenten und ihrer Lage kann eine Klassifizierung der Läsion in eine benigne oder maligne Läsion vorgenommen werden. Mittels des bekannten Verfahrens kann jedoch eine lokale Dicke des Gewebeabschnitts nicht bestimmt werden, da lediglich Oberflächeneigenschaften erfasst werden.

Die Druckschrift US 2014/324085 A1 beschreibt ein Ablationsverfahren, bei dem mittels eines Ultraschall-Transducers Energie für das Abladieren in das Gewebe eingebracht wird. Der Ultraschall-Transducer wird ferner dafür genutzt, die Größe der durch das Abladieren erzeugten Läsion zu bestimmen. Das bekannte Ablationsverfahren setzt eine aufwendige und kostenintensive Elektronik und Transducertechnik zur Erzeugung und zur Auswertung des Ultraschallsignals ein.

Die Druckschrift US 2015/209551 A1 beschreibt ebenfalls ein Ablationsverfahren mittels Ultraschall. Bei dem bekannten Verfahren wird zusätzlich mittels einer bildgebenden Spule und eines magnetisch bildgebenden Systems, mit dem die bildgebende Spule detektiert werden kann, die Position des Katheters in Bezug auf den Zielort der Behandlung bestimmt. Das bekannte Verfahren ist zu ungenau, um eine Aussage über die Tiefe der Läsion bzw. die Dicke des Gewebes zu treffen.

Aus dem Dokument US 2015/196202 A1 ist ein Verfahren zur Bestimmung einer Läsionstiefe bekannt, das auf einer Messung der mitochondrischen reduzierten Nicotinamidadenindinukleotid (NADH) - Fluoreszenzintensität des beleuchteten Herzgewebes beruht. Diese Fluoreszenzintensität kann jedoch nur zeitversetzt gemessen werden. Ebenfalls ein optisches Abfrageverfahren wird in der Druckschrift WO 2014/163974 A2 offenbart, das sich auf eine Signalgebung im einstelligen Millimeterbereich beschränkt.

Es ist wünschenswert, die Genauigkeit der Bestimmung einer lokalen Gewebeeigenschaft, z. B. der Läsionstiefe bzw. der lokalen Gewebedicke, weiter zu erhöhen. Ferner soll eine lokale Behandlung des Gewebes, beispielsweise die Ablation, durch diese Bestimmung nicht beeinträchtigt werden. Es ist weiterhin Ziel der Verbesserung, ein schnell und einfach sowie kostengünstig bestimmbares Kriterium zur Verfügung zu stellen, mit dem der Fortschritt der Behandlung beurteilt werden kann.

Die Aufgabe der vorliegenden Erfindung besteht somit darin, eine Vorrichtung zu schaffen, mit der eine lokale Gewebeeigenschaft, z. B. eine Läsionstiefe oder Gewebedicke, genau, schnell, einfach und kostengünstig sowie mit geringer Wechselwirkung mit der lokalen Gewebebehandlung bestimmt werden kann. Die Aufgabe besteht außerdem darin, ein entsprechendes einfaches Verfahren hierfür anzugeben.

Die obige Aufgabe wird gelöst durch eine Vorrichtung zur Bestimmung (Erfassung) einer lokalen Eigenschaft eines biologischen Gewebes mit

- einem flexiblen, länglichen, vorzugsweise hohlzylinderförmigen Schaft,
- einer Datenverarbeitungseinrichtung und
- einem Kernspinresonanz (NMR) -Sensor, der an dem distalen Ende des Schafts angeordnet und mit der Datenverarbeitungseinrichtung verbunden ist, wobei der NMR-Sensor ein erstes Sensorelement zur Erzeugung eines statischen Magnetfelds und ein zweites Sensorelement zur Erzeugung eines magnetischen Wechselfelds aufweist,

wobei das distale Ende des Schafts benachbart zu der zu vermessenden Stelle des Gewebes anordenbar ist, wobei die Datenverarbeitungseinrichtung zur Bestimmung einer lokalen Gewebeeigenschaft an dieser Stelle basierend auf einem zur Datenverarbeitungseinrichtung übertragenen Signal des NMR-Sensors eingerichtet ist. Aus der mittels der erfindungsgemäßen Vorrichtung bestimmten lokalen Gewebeeigenschaft (z. B. lokaler Gewebequerschnitt und/oder Art des Gewebes und/oder Anteil des Muskelgewebes und/oder Zusammensetzung des Gewebes) kann durch die Datenverarbeitungseinrichtung der Fortschritt einer Ablation bestimmt werden, beispielsweise durch Ermittlung der lokalen Tiefe der Läsion. Der NMR-Sensor ist mit dem distalen Ende des Schafts vorzugsweise nicht lösbar verbunden.

Die Erfinder haben erkannt, dass die Eigenschaften des Gewebes und ihre Änderung durch eine Ablation, insbesondere in Bezug auf Temperatur, Gewebeart (Muskelgewebe, Fettgewebe), Zusammensetzung (z. B. Wassergehalt) und/oder die Abmessungen mittels Kernspinresonanz (Nuclear Magnetic Resonance) detektiert werden können. Insbesondere die Amplitude des gemessenen Kernspinresonanzsignals kann für eine Aussage über die Größe des Läsionsgebiets, d. h. seine Abmessung, herangezogen werden.

Als lokale Gewebeeigenschaft können erfindungsgemäß beispielsweise die Temperatur der benachbarten Stelle des Gewebes, die Dicke der benachbarten Stelle des Gewebes (insbesondere in Richtung der Längsachse des Schafts), die Läsionstiefe (d. h. die Tiefe der Läsion an der benachbarten Stelle des Gewebes in Richtung der Längsachse des Schafts), die Läsionsgröße (d. h. die Abmessung der Läsion an der benachbarten Stelle des Gewebes in eine Richtung senkrecht zur Längsachse des Schafts), Informationen zum Kontakt zwischen Ablationskatheter und Gewebe (z. B. die Kompression des Gewebes oder die Kontaktkraft beruhend auf der Dichte des Gewebes), die Menge des Gewebes, welches das distale Ende des Schafts umgibt, sowie die Zusammensetzung der benachbarten Stelle des Gewebes, insbesondere sein Wassergehalt, der Anteil des Muskelgewebes an der benachbarten Stelle des Gewebes, sein Fettgehalt und/oder seine Protonendichte, bestimmt werden. Hierbei ist auch die Bestimmung mehrerer oben angegebener Gewebeeigenschaften möglich. Weiter beinhaltet erfindungsgemäß die Bestimmung einer lokalen Gewebeeigenschaft auch die Bestimmung der Veränderung der jeweiligen Gewebeeigenschaft im Verlauf der (Ablations-)Behandlung bzw. der Messung. Die zu der erfindungsgemäßen Vorrichtung benachbarte Stelle des Gewebes umfasst eine Oberfläche des Gewebes an der Stelle und ein sich an diese Oberfläche anschließender Volumenbereich dieses Gewebes, in dem, wie unten genauer beschrieben wird, eine NMR-Anregung durch den NMR-Sensor erfolgt.

Zur Erzeugung des grundsätzlich bekannten NMR-Signals werden mindestens zwei Komponenten benötigt, nämlich ein statisches Magnetfeld, nach dessen Feldlinien sich die Spins von z. B. Protonen ausrichten, und ein magnetisches Wechselfeld, durch das die Spins aus ihrem Gleichgewichtszustand angeregt werden. Das statische Magnetfeld wird durch das erste Sensorelement erzeugt, während das magnetische Wechselfeld durch das zweite Sensorelement produziert wird. Erfindungsgemäß sind das erste und das zweite Sensorelement am distalen Ende des flexiblen Schafts angeordnet, der z. B. über die Blutgefäße in den Körper eines Menschen oder Tiers eingeführt und in unmittelbarer Nachbarschaft zu der zu vermessenden Gewebestelle oder dem zu vermessenden Gewebebereich angeordnet werden kann, an dem beispielsweise die Ablation erfolgt. Voraussetzung für die Anregung sind dabei Feldkomponenten des magnetischen Wechselfeldes, die senkrecht zu den Feldlinien des statischen Feldes ausgerichtet sind. Die Frequenz, bei dem die Spins ausgelenkt werden, ist abhängig vom Betrag des magnetischen Flusses des statischen Magnetfeldes. Für die Resonanzbedingung gilt die Beziehung

$$f_L = \frac{\gamma}{2\pi} B$$

mit γ als dem gyromagnetischen Verhältnis (für Protonen $^1$H: $\gamma = 267{,}513 \times 10^6$ 1/sT) und der magnetischen Flussdichte $B$.

Nach der Anregung kann beispielsweise die Relaxationszeit der angeregten Kernspins oder der zeitliche Verlauf der Schwingungsamplitude des anregenden magnetischen Wechselfelds gemessen werden. Gewebegrenzen machen sich bei der Messung durch eine sprunghafte Änderung der genannten Messgrößen bemerkbar. Während z. B. Blut mit seinem hohem Wasseranteil eine lange Relaxationszeit aufweist, besitzen Gewebeanteile mit geringerem Wasseranteil eine vergleichsweise kurze Relaxationszeit. Diese unterschiedlichen Relaxationszeiten sind maßgeblich für den hohen Weichteilkontrast des NMR-Signals verantwortlich und ermöglichen bei einer örtlichen Kodierung der Signale mittels Magnetfeldgradienten eine örtliche Zuweisung der Gewebearten und somit der Dicke des Gewebes.

Es ist von Vorteil, wenn die NMR-Anregung durch den NMR-Sensor im Wesentlichen in einem kegelförmigen Volumen um eine Achse in Raumrichtung ausgehend vom distalen Ende des flexiblen Schafts erfolgt. Das kegelförmige Volumen ergibt sich aus dem Verlauf der magnetischen Feldlinien des statischen Magnetfeldes in Bezug auf die Feldlinien des magnetischen Wechselfeldes. Bei geeigneter Anordnung der Sensorelemente stehen die beiden Magnetfeldkomponenten hauptsächlich in einem kegelförmigen Zylinder senkrecht zueinander. Im Volumen außerhalb des Zylinders verlaufen die Magnetfeldlinien weitestgehend parallel zueinander und tragen somit nicht zum NMR-Signal bei. Der Anregungskegel hat vorzugsweise einen Öffnungswinkel (Winkel zwischen zwei gegenüber liegenden Mantellinien des Kegels) von höchstens 180°, vorzugsweise höchstens 90°. Besonders bevorzugt ist, wenn sich die Achse in distaler Richtung von dem distalen Ende des flexiblen Schafts erstreckt. Weiter von Vorteil ist, wenn sich der Anregungskegel der NMR-Anregung in Bezug auf die Oberfläche der zu vermessenden Gewebestelle so ausrichten lässt, dass sich die Achse des Anregungskegels senkrecht zu der Gewebeoberfläche an der zu vermessenden Stelle erstreckt.

Die Eindringtiefe für das NMR-Signal in das zu untersuchende Gewebe (d. h. die Höhe des Anregungskegels) ist abhängig von der Frequenz des Wechselfeldes bzw. dessen Bandbreite. Als Resonanzfrequenz ergeben sich für Protonen $^1$H Frequenzen im Megaherzbereich in unmittelbarer Nähe zum Magneten (Entfernung < 3 mm), die bis zu einer Entfernung von 35 mm auf 1 kHz abgesunken sind. Wird beispielsweise ein kugelförmiger Magnet als statischer Magnet (erstes Sensorelement) mit 1 T maximaler magnetischer Flussdichte an der Oberfläche eingesetzt, werden durch hohe Frequenzen im Megaherzbereich Spins im Abstand von bis zu 3 mm angeregt. Mit Frequenzen von 1 Mhz bis 1 kHz werden Spins in einem Abstand von bis zu 34 mm angeregt. Bei Verwendung einer geringeren magnetischen Flussdichte nimmt die Eindringtiefe in diesem Frequenzbereich entsprechend der Resonanzbedingung ab.

Mit einem derartigen breitbandigen Anregungspuls des der gewünschten Anregungstiefe entsprechenden Frequenzbereichs wird eine Volumenanregung der Spins in distaler Richtung ausgehend von dem distalen Ende des Schafts erreicht. In Abhängigkeit von ihrer Entfernung senden die angeregten Spins ein Antwortsignal mit der entsprechenden Resonanzfrequenz, sodass mittels einer Fourier-Analyse eine eindimensionale Ortsauflösung als Funktion des Abstands möglich wird.

Als Material für das erste Sensorelement zur Erzeugung eines statischen Magnetfelds wird vorzugsweise mindestens ein Material aus der Gruppe umfassend Neodym, gehärteter Stahl, Ferrite, Aluminium-Nickel-Cobalt-Legierungen, Bismut- Mangan-Eisen-Legierungen (Bismanol) oder Samarium-Cobalt-Legierungen verwendet. Die magnetischen Flussdichten an der Oberfläche des ersten Sensorelements liegen bevorzugt in einem Bereich von (einschließlich) 0,5 T bis 1,5 T.

In einem bevorzugten Ausführungsbeispiel ist das erste Sensorelement als Permanentmagnet, der beispielsweise kugelförmig oder quaderförmig gestaltet ist, oder als Spule ausgebildet. Bei Verwendung z. B. eines kugelförmigen Festkörper-Permanentmagneten (Neodym, mit beispielsweise 1 T Flussdichte an der Oberfläche) fällt die Flussdichte mit dem Abstand vom Katheter in etwa mit der dritten Potenz ab und ähnelt dem eines Stabmagneten. Die kugelförmige oder quaderförmige Gestaltung des Festkörpermagneten erlaubt eine einfache Ausrichtung des statischen Magnetfelds. Zudem kann ein gerichteter Anregungskegel erzeugt werden. In einem bevorzugten Ausführungsbeispiel ist das Material des Permanentmagneten nicht elektrisch leitfähig, um Wirbelströme, die durch das magnetische Wechselfeld induziert werden, zu vermeiden. Permanentmagneten aus z. B. Neodym und den meisten anderen Materialen haben in etwa die Permeabilität von Luft. Die Permeabilität kann aber, wie im Fall von Aluminium-Nickel-Cobalt, auch 2, 4 oder bis zu 8 betragen, wodurch die Effizienz des zweiten Sensorelements zur Erzeugung des magnetischen Wechselfeldes entsprechend erhöht wird. Um ein permanentes Magnetfeld zu erzeugen, welches sich im Vergleich zu einem Kugelmagneten weniger nichtlinear verhält, kann der Permanentmagnet auch in Form eines Hufeisenmagneten gestaltet sein, wobei vorzugsweise die Arme des Hufeisenmagneten parallel zur Längsachse des Schafts verlaufen.

Es ist weiter von Vorteil, wenn das zweite Sensorelement als Spule ausgebildet ist. Zur Erzeugung und Empfang des magnetischen Wechselfeldes werden vorzugsweise kreisförmige Leiterspulen verwendet. Im vorteilhaften Frequenzband von MHz-kHz ist eine Wicklungsanzahl von höchstens 10 bevorzugt, besonders bevorzugt eine Wicklungszahl von 5 bis 10. Bei dem Ausführungsbeispiel, bei dem das erste Sensorelement als Permanentmagnet ausgebildet ist, ist die Spule vorzugsweise um das erste Sensorelement gewickelt. Um bei einem Hufeisen-Permanentmagnet als erstes Sensorelement den Öffnungswinkel des Anregungskegels für die Signalerzeugung möglichst klein zu halten, kann die Spule für das magnetische Wechselfeld zwischen den beiden Armen des Hufeisenmagneten angeordnet sein. Vorteilhaft ist weiter die zusätzliche Verwendung eines ferromagnetischen, nicht elektrisch leitenden Spulenkerns, um die Feldstärke sowohl des ersten als auch des zweiten Sensorelements zu erhöhen. Die Feldlinien des Permanentmagneten verlaufen vorzugsweise senkrecht zur Achse des Schafts an dessen distalem Ende, die Feldlinien des magnetischen Wechselfeldes entlang der Achse des Schafts.

Die äußeren Abmessungen (Länge ggf. in Richtung der Längsachse, Breite oder Durchmesser ggf. quer zur Längsachse, ggf. Tiefe) des ersten und des zweiten Sensorelements betragen - bedingt durch den zur Verfügung stehenden Platz in/an dem distalen Ende des Schafts - zwischen 0,5 mm und 5 mm, vorzugsweise zwischen 1 mm und 3 mm.

Da die erfindungsgemäße Vorrichtung in einem Ausführungsbeispiel zur Ablation verwendet werden kann, ist es von Vorteil, wenn an dem distalen Ende des Schafts eine elektrische leitende Fläche in Form einer metallisierten Schaftspitze angeordnet ist. Mittels der Schaftspitze wird elektrischer Strom in das benachbarte Gewebe eingebracht, was eine Gewebeläsion erzeugt. In ihrer Funktion als Ablationsfläche schirmt diese Metallfläche elektromagnetische Wellen, insbesondere deren magnetischen Anteil, ab. Um die Metallfläche für magnetische Felder und somit für die Erzeugung eines NMR-Signals und insbesondere für den Empfang von NMR-Signalen durchlässig zu gestalten, ist mindestens eine schlitzförmige durchgehende Ausnehmung in der metallischen Schaftspitze, z. B. in Form eines Kreuzschlitzes, vorgesehen, um die Ausbildung von Wirbelströmen in der Schaftspitze zu vermeiden. In einem alternativen Ausführungsbeispiel ist die Schaftspitze als Helixantenne gestaltet, um den beschriebenen Abschirmeffekt zu verringern. Je nach Ausrichtung der magnetischen Feldlinien des statischen Magnetfeldes muss die schlitzförmige Ausnehmung der Helixantenne derart gestaltet sein, dass die magnetischen Feldlinien des Wechselfeldes im gewünschten Anregungsgebiet senkrecht zu den Feldlinien des statischen Magnetfelds des ersten Sensorelements verlaufen.

Ein einfaches Ausführungsbeispiel für einen NMR-Sensor ergibt sich aufgrund der geometrischen Randbedingungen des Schafts, wenn als zweites Sensorelement eine Spule zur Erzeugung des magnetischen Wechselfeldes um z. B. einen kugelförmigen Permanentmagneten als erstes Sensorelement gewickelt wird, der das statische Magnetfeld erzeugt.

In einem Ausführungsbeispiel der Erfindung ist der NMR-Sensor mittels mindestens eines an dem NMR-Sensor befestigten Zugseils gegenüber dem Schaft mittels eines entsprechenden Steuermechanismus schwenkbar und/oder drehbar, um die Achse des Anregungskegels des NMR-Sensors in eine Richtung senkrecht zur Oberfläche des Gewebes mit der zu untersuchenden Gewebestelle auszurichten. Das mindestens eine Zugseil ist bevorzug an dem äußeren Umfang des ersten Sensorelements befestigt. Insbesondere die Geometrie des NMR-Sensors mit einem kugelförmigen Permanentmagneten als erstes Sensorelement erlaubt die Drehung der Kombination von Permanent- und Elektromagnet in die Richtung, die von besonderem Interesse für die Signalgebung ist, wenn z. B. der Schaft an seinem distalen Ende in einem vergleichsweise flachen Winkel in Bezug auf die Gewebeoberfläche angeordnet ist. In Ausführungsbeispielen können zwei gegenüber liegend angeordnete Zugseile (d.h. in einem Winkel von 180° voneinander beabstandet) oder jeweils im Winkel von 90° beabstandete Zugseile vorgesehen sein, die vorzugsweise durch den Schaft hindurchgehen und von außen betätigt werden können, um den NMR-Sensor in Bezug auf die Längsachse des Schafts zu schwenken und/oder zu drehen. Zusätzlich kann der Schaft um seine Längsachse gedreht werden.

In einem weiteren Ausführungsbeispiel kann der NMR-Sensor auf einer Unterlage gelagert sein, welche einen ersten Abschnitt mit einer hohen oder höheren Elastizität, vorzugsweise in Richtung der Längsachse des Schafts, und einen zweiten Abschnitt mit einer verglichen mit dem ersten Abschnitt geringeren Elastizität besitzt. Der erste Abschnitt und der zweite Abschnitt sind vorzugsweise in eine Richtung quer zur Längsachse des Schafts nebeneinander angeordnet. Bei einem Verschwenken des NMR-Sensors in Bezug auf die Längsachse des Schafts bewirkt der erste Abschnitt eine Rückstellkraft. Hierdurch wird die Ausrichtung des NMR-Sensors erleichtert und die Vorrichtung einfacher gestaltet, da nur ein einziges Zugseil erforderlich ist. Der zweite Abschnitt der Unterlage mit der geringeren Elastizität (oder höheren Steifigkeit) kann z. B. aus einem Kunststoff wie beispielsweise TPU (thermoplastisches Polyurethan), PEEK ,(Polyetheretherketon), Polyetherblockamid (PEBA, wie z.B. Pebax) oder LCP (Flüssigkristallpolymer, liquid crystral polymer) bestehen. Der erste Abschnitt der Unterlage mit der hohen oder höheren Elastizität kann z. B. aus einem aufgeschäumten Kunststoff oder Silikon bestehen oder eine blattfederartigen Struktur besitzt, die beispielsweise aus einem Kunststoff gefertigt ist.

Aufgrund des vergleichsweise starken und nichtlinearen statischen Magnetfeldgradienten eines kugelförmigen Festkörpermagneten werden die angeregten Spins in kurzer Zeit dephasieren und eine Aufnahme der Signalantwort entsprechend erschwert. Diesem Umstand lässt sich durch die Anregung mittels magnetischer Wechselfeldpulse durch den NMR-Sensor und mittels Verwendung eines Spinecho entgegenwirken, indem z. B. nach einem 90° Anregungspuls ein weiterer Puls gesendet wird, der die Spins um 180° dreht, also umkehrt. Die Dauer eines Wechselfeldpulses beträgt zwischen 1 und 50 Millisekunden, vorzugsweise zwischen 1 und 20 Millisekunden.

Die obige Aufgabe wird mit analogen Vorteilen auch durch einen Katheter aufweisend eine oben beschriebene Vorrichtung gelöst. Neben der Bestimmung der Gewebeeigenschaft können weitere in oder an dem Katheter angeordnete Komponenten bzw. mit dem Katheter verbundene Komponenten die Positionierung an einem geeigneten Therapieort erleichtern. Derartige Komponenten können beispielsweise eine Einrichtung zur Navigation sein, wobei der Katheter in diesem Fall z. B. mit einem Magnetometer oder einem Elektrofeldmeter verbunden. Das extrakorporal von dem Magnetometer oder dem Elektrofeldmeter erzeugte Feld zur Positionsbestimmung ist dabei so abgestimmt, dass es das NMR-Signal nicht beeinflusst. Weitere Komponenten am Katheter zur Positionierung an einem geeigneten Therapieort sind an dem Katheter angeordnete Elektroden in Form von Ringelektroden oder Minielektroden, die das Erfassen lokaler elektrischer Signale ermöglichen. Damit können lokale Zellaktivitäten im Kontext der Läsionsbildung und dem Reizleitungssystem bewertet werden. Als eine weitere Komponente zur Kontrolle der Läsionserzeugung können an dem Katheter ein Kraftsensor oder mehrere Kraftsensoren angeordnet sein (beispielsweise an dem distalen Ende des Schafts), deren Umformer gewöhnlich auf elektromagnetischen oder faseroptischen Prinzipien beruht. Die elektromagnetische Wechselwirkung des oder der entsprechenden Komponenten mit dem NMR-Sensor muss berücksichtigt werden. So können z. B. die Frequenzen der elektromagnetischen Felder abgestimmt, die Störfelder während der Messung abgeschaltet oder entsprechende Filter bzw. Signalaufbereitungselemente eingesetzt werden. Bei Integration des zweiten Sensorelements in eine an dem distalen Ende des Schafts angeordnete Ablationselektrode kann das zweite Sensorelement zur Energieabgabe bei Ablation mit genutzt werden, wodurch die Energieabgabe optimiert wird.

Die obige Aufgabe wird auch durch ein Verfahren zur Bestimmung einer lokalen Eigenschaft eines biologischen Gewebes gelöst, bei dem nach Anregung durch einen benachbart zu der zu vermessenden Stelle des Gewebes an dem distalen Ende eines flexiblen Schafts angeordneten NMR-Sensor ein NMR-Antwortsignal (im Folgenden kurz NMR-Signal) des Gewebes erzeugt und basierend auf diesem NMR-Signal die lokale Gewebeeigenschaft bestimmt wird. Die Auswertung des NMR-Signals entspricht prinzipiell der Auswertung bildgebender MRT-Signale. Die empfangenen NMR-Signale werden in der Datenverarbeitungseinrichtung sowohl über ihre Amplitude als auch ihre Phase charakterisiert. Über die Phase ist es möglich, die Temperaturänderung über die Zeit zu quantifizieren. Die Amplitude wird bestimmt durch die Protonendichte des Gewebes und die, für Gewebearten charakteristische, Quer- (T2-) und Längs- (T1-)Relaxationszeiten. Die TI-Zeit ist zudem von der Temperatur des Gewebes abhängig. Eine Erhöhung der Temperatur erhöht gleichzeitig die T1-Relaxationszeit des betroffenen Areals, was unmittelbar zu einer Reduzierung des NMR-Signals führt. Das Auftreten einer Läsion durch die Einbringung von thermischer Energie ändert mittelfristig den Wasseranteil des Gewebes, was zu einer Änderung der Dichte der freien Protonen und einer Änderung der T2-Relaxationszeit führt.

Das erfindungsgemäße Verfahren besitzt die oben zur Vorrichtung erläuterten Vorteile. Die Steuerung der Anregung mittels NMR-Sensor und die Bestimmung der lokalen Gewebeeigenschaft aus den übermittelten NMR-Signalen erfolgt mittels der Datenverarbeitungseinrichtung.

Zu den mit dem erfindungsgemäßen Verfahren bestimmbaren lokalen Eigenschaften des biologischen Gewebes wird auf die obigen Erläuterungen zur erfindungsgemäßen Vorrichtung verwiesen.

Wie oben bereits beschrieben wurde, wird in einem Ausführungsbeispiel des erfindungsgemäßen Verfahrens vor der Erzeugung des NMR-Signals die Achse eines Anregungskegels des NMR-Sensors im Wesentlichen senkrecht zur Gewebeoberfläche ausgerichtet. Besonders bevorzugt erfolgt die Ausrichtung

- durch Betätigen mindestens eines an dem NMR-Sensor befestigten Zugseils, beispielsweise mittels eines am Schaft angeordneten Steuermechanismus, so dass ein Schwenken und/oder Drehen des NMR-Sensors in Bezug auf die Längsachse des Schafts bewirkt wird, und/oder

- durch Drehen des Schafts. Zusätzlich oder alternativ kann das distale Ende des Schafts derart in Richtung der Längsachse des Schafts verschoben werden, dass das distale Ende des Schafts an der Oberfläche des zu vermessenden Gewebes anliegt.

In einem weiteren Ausführungsbeispiel wird intermittierend zwischen der Bestimmung der lokalen Gewebeeigenschaft basierend auf dem NMR-Signal eine an dem distalen Ende des Schafts angeordnete Schaftspitze mit einem Strom versorgt oder an der metallischen Schaftspitze eine Spannung angelegt, so dass mittels der Schaftspitze eine Ablation des Gewebes und die Erzeugung einer Läsion in dem Gewebe erfolgt.

In einem weiteren Ausführungsbeispiel des erfindungsgemäßen Verfahrens erfolgt, wie oben erläutert wurde, die Anregung mittels magnetischer Wechselfeldpulse durch den NMR-Sensor und mittels Verwendung eines Spinecho-Verfahrens, indem z. B. nach einem Anregungspuls (wird auch als 90°-Anregungspuls bezeichnet) ein weiterer Puls gesendet wird, der die Spins um 180° dreht.

Die obige Aufgabenstellung wird ferner gelöst durch ein Computerprogrammprodukt zur Bestimmung einer lokalen Eigenschaft eines biologischen Gewebes mit Programmcodemitteln zur Ausführung eines Computerprogramms nach dessen Implementierung in einer Datenverarbeitungseinrichtung. Die Programmcodemittel sind dazu vorgesehen, nach der Implementierung in der Datenverarbeitungseinrichtung das oben beschriebene Verfahren auszuführen. Das erfindungsgemäße Computerprogrammprodukt besitzt die oben zum erfindungsgemäßen Verfahren erläuterten Vorteile.

Die Erfindung wird nachfolgend anhand von Ausführungsbeispielen und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:

Fig. 1    einen erfindungsgemäßen Katheter in einer Ansicht von der Seite,

Fig. 2    eine erfindungsgemäße Vorrichtung in einer Ansicht von der Seite,

Fig. 3    ein erstes Ausführungsbeispiel für die prinzipielle Realisierung des NMR-Sensors der Vorrichtung gemäß Fig. 2,

Fig. 4    ein zweites Ausführungsbeispiel für die prinzipielle Realisierung des NMR-Sensors der Vorrichtung gemäß Fig. 2,

Fig. 5 ein drittes Ausführungsbeispiel für die prinzipielle Realisierung des NMR-Sensors der Vorrichtung gemäß Fig. 2,

Fig. 6 ein zweites Ausführungsbeispiel einer erfindungsgemäßen Vorrichtung in einer Ansicht von der Seite einschließlich der Magnetfeldlinien des ersten Sensorelements,

Fig. 7 den NMR-Sensor der Vorrichtung gemäß Fig. 6 in einer Ansicht von der Seite,

Fig. 8 die Schaftspitze der Vorrichtung gemäß Fig. 6 einschließlich der Magnetfeldlinien des zweiten Sensorelements in einer Ansicht von der Seite,

Fig. 9 ein zweites Ausführungsbeispiel einer Schaftspitze der Vorrichtung gemäß Fig. 6 in einer Ansicht von oben,

Fig. 10 ein drittes Ausführungsbeispiel einer Schaftspitze der Vorrichtung gemäß Fig. 6 in einer Ansicht von der Seite,

Fig. 11 die Schaftspitze gemäß Fig. 10 in einer Ansicht von oben,

Fig. 12 ein drittes Ausführungsbeispiel einer erfindungsgemäßen Vorrichtung in einer Ansicht von der Seite einschließlich der Magnetfeldlinien des ersten Sensorelements,

Fig. 13 den NMR-Sensor der Vorrichtung gemäß Fig. 12 in einer Ansicht von der Seite,

Fig. 14 die Schaftspitze der Vorrichtung gemäß Fig. 12 einschließlich der Magnetfeldlinien des zweiten Sensorelements in einer Ansicht von der Seite,

Fig. 15 ein zweites Ausführungsbeispiel einer Schaftspitze der Vorrichtung gemäß Fig. 12 in einer Ansicht von der Seite,

Fig. 16 die Schaftspitze gemäß Fig. 15 in einer Ansicht von oben,

Fig. 17 - 22 die Ausrichtung des Anregungskegels mittels Drehen des NMR-Sensors der Vorrichtung gemäß Fig. 9,

Fig. 23 ein weiteres Ausführungsbeispiel für einen NMR-Sensor einer erfindungsgemäßen Vorrichtung in einer Ansicht von der Seite,

Fig. 24 die Magnetfeldlinien des zweiten Sensorelements des NMR-Sensors gemäß Fig. 23,

Fig. 25 die Magnetfeldlinien des ersten Sensorelements des NMR-Sensors gemäß Fig. 23,

Fig. 26 - 27 die Ausrichtung des Anregungskegels mittels schwenken des NMR-Sensors gemäß Fig. 23,

Fig. 28 die Anregung der Protonen mittels des NMR-Sensors nach der Spinecho-Methode in der Zeitdomäne und der Frequenzdomäne.

Der Aufbau und die Arbeitsweise eines erfindungsgemäßen Katheters bzw. einer erfindungsgemäßen Vorrichtung mit einem Schaft werden nachfolgend anhand eines Ablations-Katheters erläutert, der für die intrakardiale Ablation eingesetzt wird. Die vorliegende Erfindung soll jedoch hierauf nicht beschränkt werden. Der Aufbau und die Arbeitsweise eines erfindungsgemäßen Katheters bzw. einer erfindungsgemäßen Vorrichtung kann analog auch auf Katheter/Vorrichtungen für andere Behandlungen bzw. andere Gewebe übertragen werden, bei denen die Bestimmung der lokalen Gewebeeigenschaft, z. B. der lokalen Dicke bzw. lokalen Läsionstiefe, von Bedeutung ist.

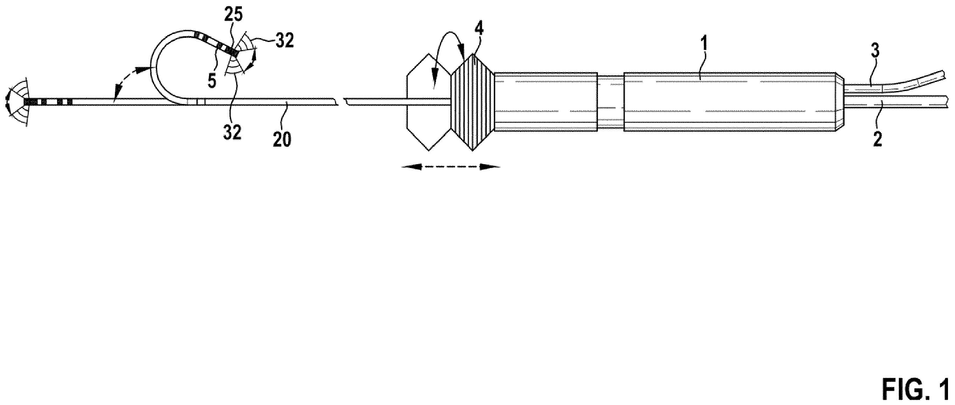

Fig. 1 zeigt ein Ausführungsbeispiel eines erfindungsgemäßen Katheters mit einem Griff 1, mindestens einer elektrischen und/oder optischen Signalleitung 2 für die Übermittlung von Signalen von und/oder zu dem mindestens einen an dem Katheter angebrachten Sensor oder Sensorelement und/oder der mindestens einen Elektrode, einer Spülleitung 3, einem Steuermechanismus 4 und einem Innenschaft 20. Der Innenschaft 20 ist Bestandteil der erfindungsgemäßen Vorrichtung. Für eine Ablation wird der Innenschaft 20 so weit in den Körper des Patienten eingeführt, beispielsweise entlang der Blutgefäße des Patienten, bis das distale Ende des Innenschafts 20 an der gewünschten Stelle des Herzmuskelgewebes, an der abladiert werden soll, anliegt. Zum Erfassen der elektrischen Herzaktivität ist mindestens eine Elektrode 5 am distalen Ende des Innenschafts 20 vorgesehen. In der in Fig. 1 dargestellten Ausführungsform ist die Elektrode 5 als Ringelektrode ausgebildet. Eine innerhalb der distalen Spitze des Innenschafts 20 angeordnete Minielektrode ist ebenfalls denkbar. Mittels des Steuermechanismus 4 kann das distale Ende des Innenschafts 20 beispielsweise über einen Push-Pull-Mechanismus deflektiert werden, wie dies mittels der gestrichelten Pfeile veranschaulicht ist. Zusätzlich kann, wie unten genauer beschrieben wird, eine Ausrichtung des Anregungskegels 32 zu dem zu untersuchenden und zu abladierenden Gewebe mittels einer Drehbewegung am Steuermechanismus 4 vorgenommen werden. Alternativ zu der händischen Steuerung mittels Steuermechanismus 4 ist eine bidirektionale oder automatisierte Steuerung anwendbar.

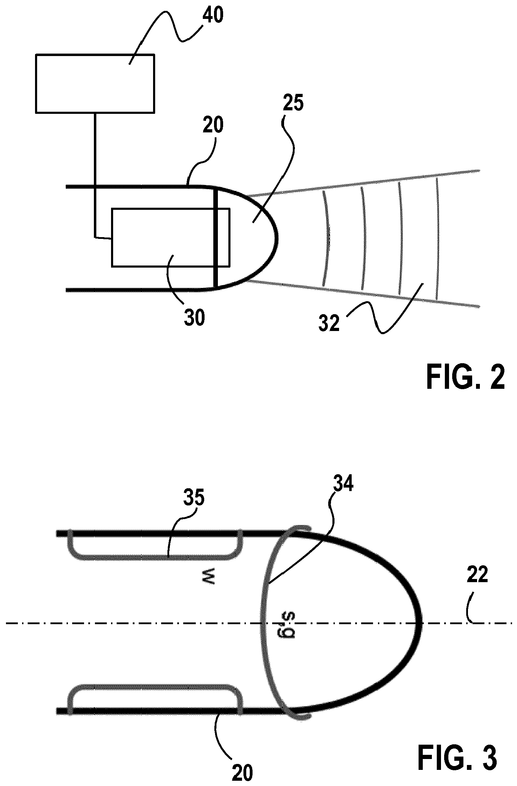

An dem distalen Ende des Innenschafts 20 (siehe Fig. 2) ist eine elektrisch leitende Schaftspitze 25 vorgesehen, die an einen elektrischen Stromkreis angeschlossen ist. Die Anschlüsse liegen auf der Innenseite der Schaftspitze 25 und sind durch den Innenschaft 20 hindurch geführt. Für die Ablation wird die Schaftspitze 25 über eine Signalleitung 2 mit einem elektrischen Hochfrequenzstrom beaufschlagt. Durch den Kontakt der Schaftspitze 25 gelangt der Hochfrequenzstrom auch in das an der Schaftspitze 25 anliegende Herzmuskelgewebe, das hierdurch verödet wird.

Um den Fortschritt der Ablation zu beurteilen, besitzt der erfindungsgemäßen Katheter an dem distalen Ende des Innenschafts 20 einen NMR-Sensor 30. Dieser NMR-Sensor 30 ist mit einer außerhalb des Körpers des Patienten angeordneten Datenverarbeitungseinrichtung 40 (beispielsweise einem (Mikro-)Prozessor oder einem Computer) verbunden. Die Beurteilung des Fortschritts der Ablation erfolgt durch den NMR-Sensor 30 und wird durch die Datenverarbeitungseinrichtung 40 gesteuert. Vor Beginn der Behandlung und nach jedem Behandlungsschritt wird der NMR-Sensor 30 durch die Datenverarbeitungseinrichtung 40 aktiviert und regt in einem Anregungskegel 32 die Protonen des in dem Anregungskegels 32 liegenden Herzmuskelgewebes 50 an. Durch die Überlagerung eines statischen Magnetfelds und eines magnetischen Wechselfelds werden die Spins der Protonen ausgerichtet und aus ihrem Gleichgewichtszustand gebracht. Das durch die Rückkehr der Protonen in den Gleichgewichtszustand von diesen ausgesandte NMR-Signal wird durch den NMR-Sensor 30 detektiert und an die Datenverarbeitungseinrichtung 40 übertragen. Diese berechnet aus dem Unterschied zwischen der Amplitude und Phase des NMR-Signals vor Beginn der Ablation und dem zuletzt gemessenen NMR-Signal, insbesondere dem Unterschied in der Amplitude, beispielsweise die Abnahme der Dicke des Herzmuskelgewebes an der im Anregungskegel 32 liegenden Stelle und hieraus die Läsionstiefe. Sobald eine ausreichende Läsionstiefe erreicht ist, kann die Behandlung an dieser Stelle abgeschlossen und gegebenenfalls an anderer Stelle fortgesetzt werden. Der Grenzwert für die Amplituden- und/oder Phasenänderung des NMR-Signals, bei dem die Behandlung abgebrochen wird, kann experimentell ermittelt werden.

Der erfindungsgemäße Katheter ermöglicht somit auf einfache Weise eine genaue Beurteilung des Fortschritts der Ablation.

Wie bereits oben erläutert wurde, besitzt der NMR-Sensor 30 ein erstes Sensorelement 34, das ein statisches Magnetfeld erzeugt, und ein zweites Sensorelement 35, das ein magnetisches Wechselfeld produziert. Hierbei müssen zumindest im Anregungskegels 32 die Feldlinien des statischen Magnetfelds des ersten Sensorelements 34 und die Feldlinien des magnetischen Wechselfelds des zweiten Sensorelements 35 senkrecht zueinander angeordnet sein. Anhand der Figuren 3 bis 5 sind 3 prinzipielle Ausführungsbeispiele für die Realisierung des ersten und des zweiten Sensorelements dargestellt.

In dem Ausführungsbeispiel gemäß Fig. 3 ist das erste Sensorelement 34 als Spule gestaltet, deren Magnetfeldlinien parallel zur (Längs-)Achse 22 des Innenschafts 20 verlaufen. Das zweite Sensorelement 35 ist ebenfalls als Spule gestaltet, wobei die Magnetfeldlinien dieser Spule senkrecht zur Achse 22 verlaufen. In einem alternativen Ausführungsbeispiel können sowohl das erste Sensorelement 34 als auch das zweite Sensorelement 35 jeweils als Spule ausgeführt sein, wobei in diesem Fall die Magnetfeldlinien des ersten Sensorelements senkrecht zur Achse 22 des Innenschafts 20 und die Magnetfeldlinien des zweiten Sensorelement parallel zur Achse 22 des Innenschafts 20 verlaufen.

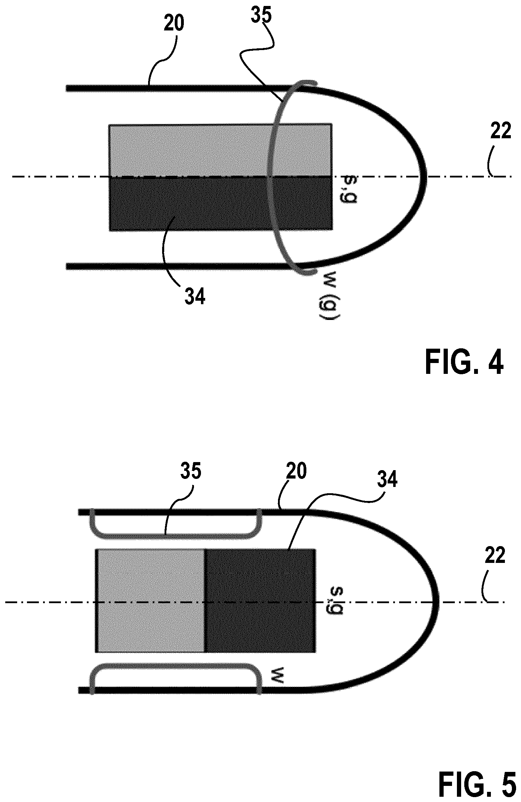

In den in den Figuren 4 und 5 dargestellten Ausführungsbeispielen ist jeweils das erste Sensorelement 34 als Permanentmagnet gestaltet. Demgegenüber ist jeweils das zweite Sensorelement 35 als Spule ausgeführt. Bei dem in Fig. 4 gezeigten Ausführungsbeispiel verlaufen die Magnetfeldlinien des ersten Sensorelements 34 senkrecht zur Achse 22 des Innenschafts 20 und bei dem in Fig. 5 gezeigten Ausführungsbeispiel parallel zur Achse 22 des Innenschafts 20. Entsprechend verlaufen die Magnetfeldlinien des zweiten Sensorelements 35 bei dem in Fig. 4 gezeigten Ausführungsbeispiel parallel und bei dem in Fig. 5 gezeigten Ausführungsbeispiel senkrecht zur Achse 22 des Innenschafts 20.

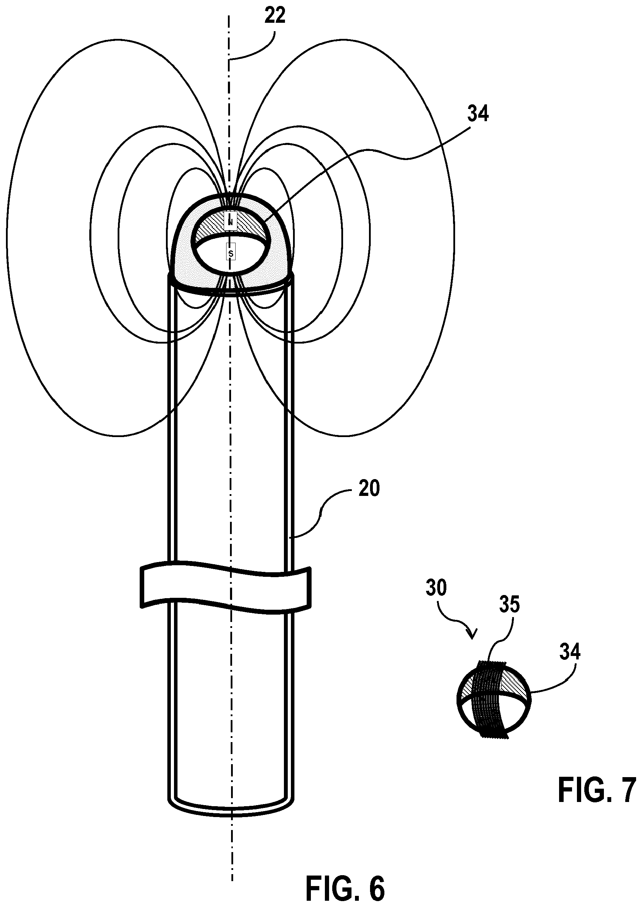

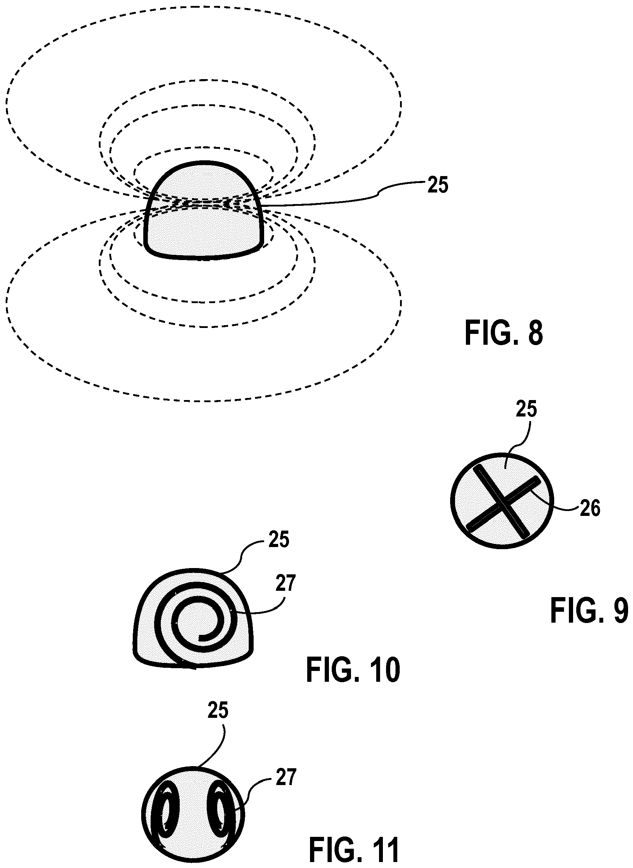

Das in den Figuren 6 und 7 gezeigte Ausführungsbeispiel entspricht dem in Fig. 5 gezeigten Prinzip, wobei das erste Sensorelement 34 kugelförmig gestaltet ist. Das zweite Sensorelement 35 ist eine Spule, die um das kugelförmige erste Sensorelement gewickelt ist, das beispielsweise aus Neodym gefertigt ist. Die Anordnung aus erstem Sensorelement 34 und zweitem Sensorelement 35 ist in Fig. 7 gezeigt. Das erste Sensorelement hat beispielsweise einen Durchmesser von 2 mm. Die magnetische Flussdichte des ersten Sensorelements beträgt beispielsweise 1 T an der Oberfläche. Die Magnetfeldlinien des ersten Sensorelements 34 sind in Fig. 6 eingezeichnet, während die Magnetfeldlinien des zweiten Sensorelements in Fig. 8 gezeigt sind (siehe gestrichelte Linien).

Um die Ausbildung von abschirmenden Kreisströmen in der metallischen Schaftspitze 25 zu vermeiden weist diese einen Kreuzschlitz 26 auf, der durch die Schaftspitze 25 hindurch geht. Der Schlitz des Kreuzschlitz hat beispielsweise eine Breite von 0,1 mm (vergleiche Fig. 9). Alternativ ist seitlich an der Schaftspitze 25 ein durchgehender spiralförmiger Schlitz 27 vorgesehen. Die Achse der Spirale verläuft, wie Figuren 10 und 11 zu entnehmen ist, in einem Winkel von mindestens 70° zur Achse 22 des Innenschafts 20. Der spiralförmige Schlitz 27 hat ebenfalls beispielsweise eine Breite von 0,1 mm.

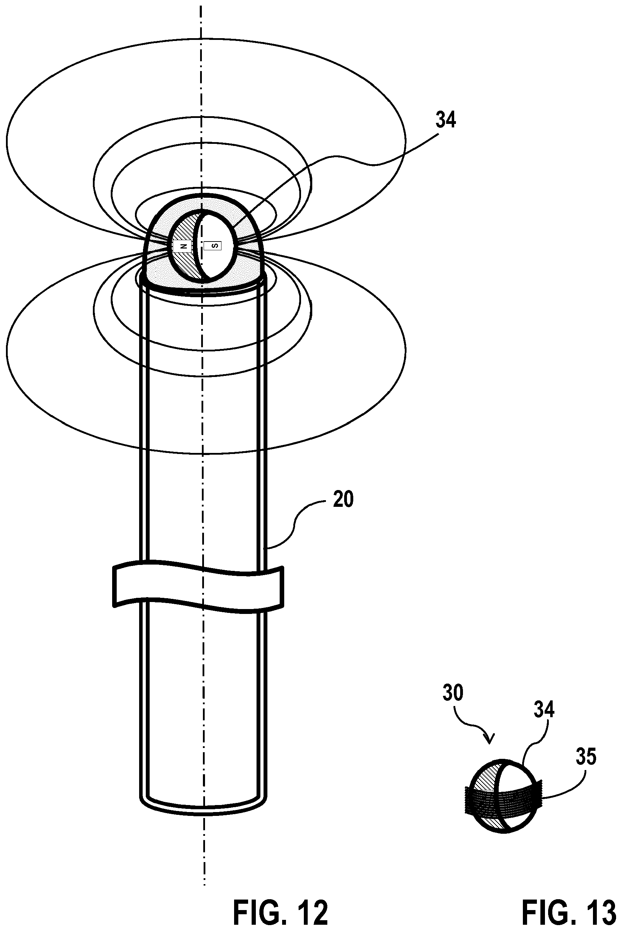

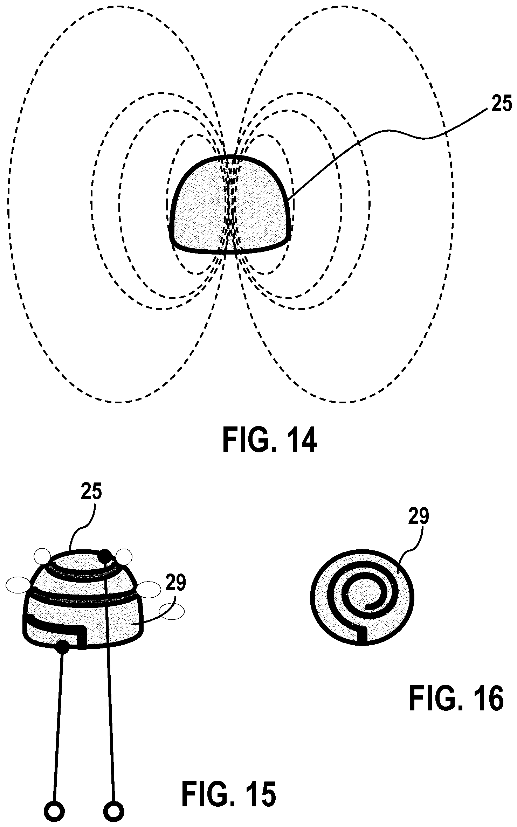

Das in den Figuren 12 und 13 gezeigte Ausführungsbeispiel entspricht dem in Fig. 4 gezeigten Prinzip der Anordnung des ersten und des zweiten Sensorelements, wobei auch bei diesem Ausführungsbeispiel das erste Sensorelement 34 als kugelförmiger Neodym-Permanentmagnet ausgebildet ist. Das zweite Sensorelement 35 ist eine Spule, die um das kugelförmige erste Sensorelement 34 gewickelt ist. Die Anordnung aus erstem Sensorelement 34 und zweitem Sensorelement 35 ist in Fig. 13 gezeigt. Das erste Sensorelement hat beispielsweise einen Durchmesser von 2 mm. Die magnetische Flussdichte des ersten Sensorelements 34 beträgt beispielsweise 1 T an der Oberfläche. Die Magnetfeldlinien des ersten Sensorelements 34 sind in Fig. 12 eingezeichnet, während die Magnetfeldlinien des zweiten Sensorelements 35 in Fig. 14 gezeigt sind (siehe gestrichelte Linien).

Um bei dem in Fig. 12 gezeigten Ausführungsbeispiel die Ausbildung von abschirmenden Kreisströmen in der metallischen Schaftspitze 25 zu vermeiden, ist diese, wie in den Figuren 15 und 16 gezeigt, als Helixantenne 29 gestaltet. Die Anzahl der Helixwindungen ist durch die Länge der metallischen Katheterspitze limitiert und liegt vorzugsweise im Bereich von 5 bis 10 Windungen. Im Bereich der sich verjüngenden Katheterspitze können die Windungen der Helixantenne 29 zur Erhöhung der Bandbreite der Antenne äquiangular oder äquidistant (Archimedische Spirale) ausgebildet sein. Die Dicke des Drahts der Helixantenne beträgt beispielsweise zwischen 0,05 mm und 0,5 mm.

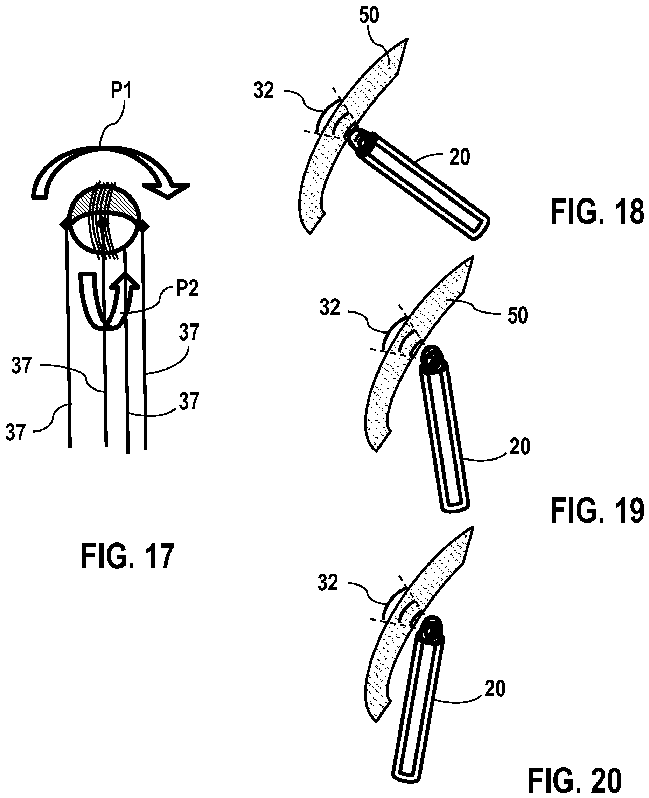

Um den NMR-Sensor 30 des in Fig. 6 gezeigten Ausführungsbeispiels so auszurichten, dass die Achse des Anregungskegels 32 etwa senkrecht zur Oberfläche des Herzmuskelgewebes an der zu untersuchenden Stelle verläuft, sind am Umfang des ersten Sensorelements 34 vier Zugseile 37 befestigt. Dies ist in Fig. 17 dargestellt. Die vier Zugseile 37 sind derart am Umfang des ersten Sensorelements 34 angeordnet, dass sie mit dem jeweils benachbarten Zugseil 37 einen Winkel von 90° einschließen. Durch entsprechendes Ziehen an einem oder mehreren Zugseilen 37 kann der beweglich gelagerte NMR-Sensor 30 um den Mittelpunkt oder einen anderen, vorzugsweise auf der Achse 22 des Innenschafts 20 liegenden Punkt innerhalb des ersten Sensorelements 34 und somit in Bezug auf die Achse 22 gedreht und/oder geschwenkt werden (siehe Pfeile P1 und P2). Die Lagerung des NMR-Sensors 30 kann z. B. mittels eines (nicht dargestellten) Kugelschalensegments erfolgen, wobei der NMR-Sensor in dem Kugelschalensegment angeordnet ist. Beispiele für eine derartige Ausrichtung in Bezug auf das Herzmuskelgewebe 50 sind in den Figuren 18 bis 20 gezeigt. Bei der Variante der Fig. 18 verläuft der Anregungskegel 32 im Wesentlichen parallel zur Achse 22 des Innenschafts 20. Bei der Konstellation der Fig. 19 verläuft die Achse des Anregungskegels 32 beispielsweise unter einem Winkel von 30° zu der Achse des Anregungskegels 32. Fig. 20 zeigt, dass durch diese Handhabung der Anregungskegel 32 so in Bezug auf die Achse des Innenschafts 20 geschwenkt werden kann, dass dessen Achse mit der Achse 22 des Innenschafts etwa 70° einschließt.

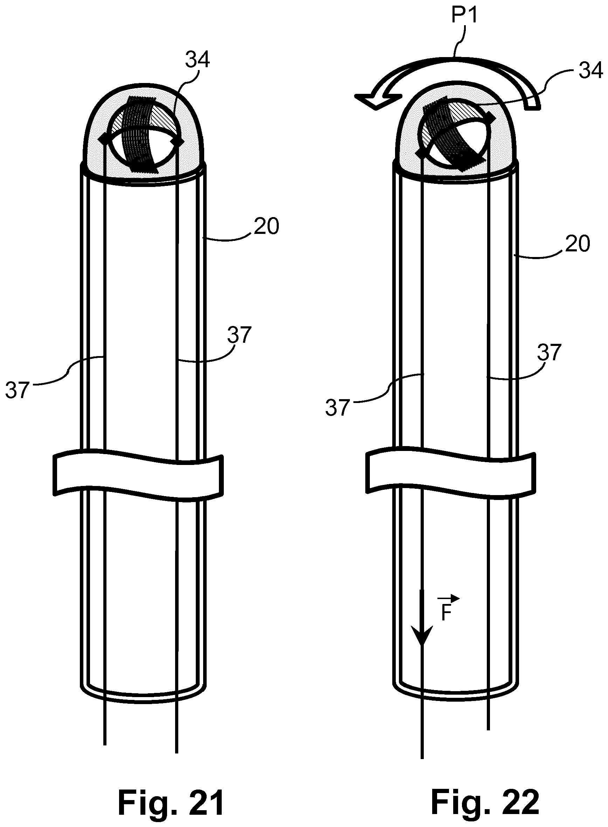

Eine ähnliche Handhabung kann auch mittels einer Anordnung erreicht werden, bei der lediglich zwei Zugseile 37 vorgesehen sind, die am Umfang des ersten Sensorelements 34 befestigt sind, und zwar einander gegenüber liegend. Ein solches Ausführungsbeispiel ist in den Figuren 21 und 22 dargestellt. Der an einem Zugseil 37 angeordnete Pfeil F stellt die Kraft (Betrag und Richtung) dar, die durch Ziehen an dem Zugseil 37 zum Drehen oder Verschwenken des NMR-Sensors 30 (siehe Pfeil P1) zu der Achse 22 aufgebracht wird. Um die Ausrichtung des Anregungskegels 32 in eine beliebige (dreidimensionale) Richtung zu erreichen, kann zusätzlich der Innenschaft 20 um seine Achse 22 gedreht werden.

Die Bewegung des Anregungskegels erfolgt vorzugsweise mittels des Steuermechanismus 4.

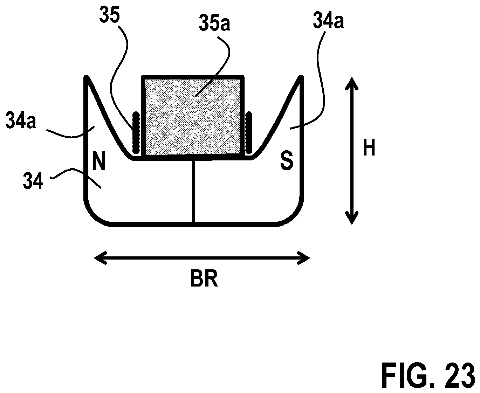

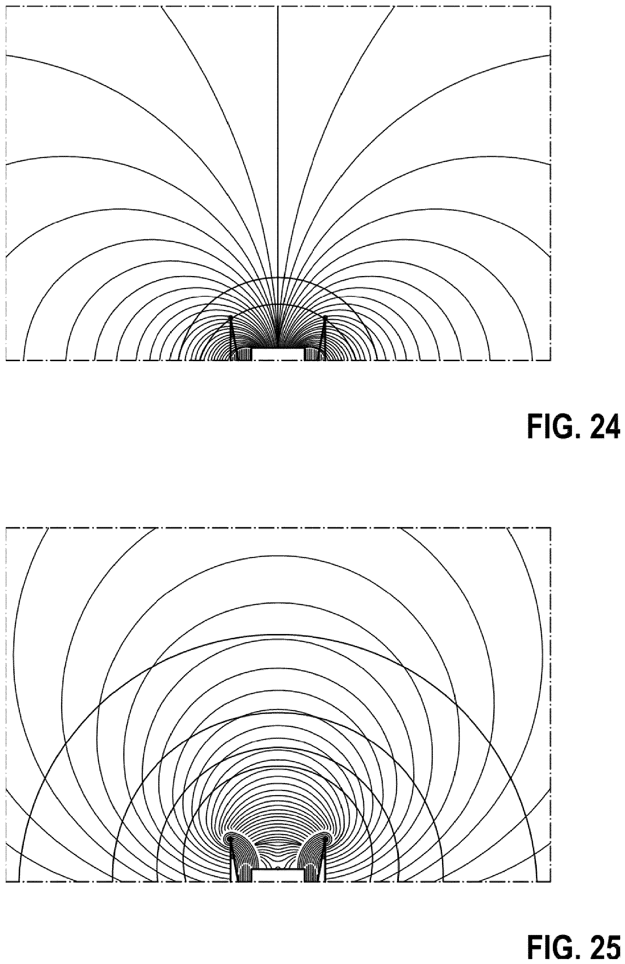

Fig. 23 zeigt ein weiteres Ausführungsbeispiel für einen NMR-Sensor 30, der sich im Vergleich mit den oben beschriebenen Ausführungsbeispielen mit dem kugelförmigen Permanentmagneten weniger nichtlinear verhält. Das erste Sensorelement 34 wird durch einen hufeisenförmigen Permanentmagneten, welcher vorzugsweise aus Neodym gefertigt ist, gebildet. Das erste Sensorelement 34 besitzt beispielsweise eine Breite B der Basis von 2 mm und eine Höhe H der Arme 34a von 1 mm bis 2 mm. Die Magnetfeldlinien des ersten Sensorelements sind in Fig. 25 dargestellt und verlaufen senkrecht zur Achse 22 des Innenschafts 20. Um den Öffnungswinkel des Anregungskegels 32 möglichst klein zu halten, ist das zweiten Sensorelement 35 als Spule gestaltet, welche zwischen den Armen 34a des hufeisenförmigen ersten Sensorelements 34 angeordnet ist. In einem bevorzugten Ausführungsbeispiel weist das zweite Sensorelement 35 einen ferromagnetischen, nicht elektrisch leitenden Spulenkern 35a auf, der die erzielte Feldstärke erhöht. Die Magnetfeldlinien des zweiten Sensorelements 35 sind in Fig. 24 eingezeichnet und verlaufen parallel zur Achse 22 des Innenschafts 20.

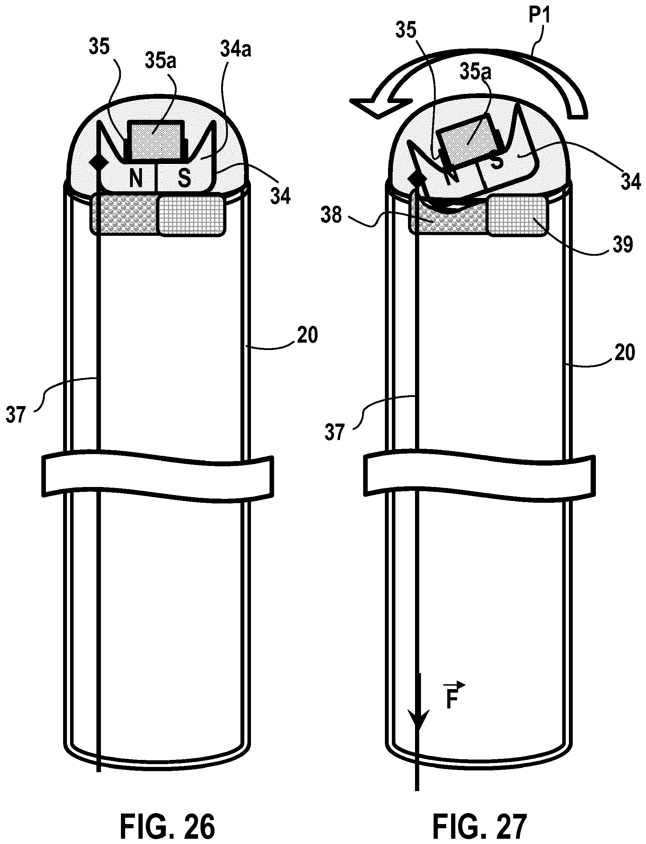

Wie in den Fig. 26 und 27 gezeigt ist, wird der NMR-Sensor 30 auf einer zumindest bereichsweise elastischen Unterlage gelagert. Die Unterlage weist einen ersten Abschnitt 38, der eine höhere Elastizität besitzt, und einen zweiten Abschnitt 39, der eine geringere Elastizität besitzt auf, wobei der erste Abschnitt 38 und der zweite Abschnitt 39 quer zur Längsachse des Innenschafts 20 nebeneinander angeordnet sind. Ferner ist an der Außenseite eines Arms 34a des ersten Sensorelements 34 ein Zugseil 37 befestigt. Durch Ziehen an dem Zugseil (siehe durch Pfeil angedeutete Richtung der Kraft F in Fig. 27), beispielsweise mittels des Steuermechanismus 4, wird der NMR-Sensor um eine senkrecht auf dem Bild der Fig. 27 stehende Achse (vgl. Pfeil P1) und somit auch zu der Längsachse des Schafts 20 geschwenkt, so dass der Anregungskegel in Bezug auf eine Gewebeoberfläche ausgerichtet werden kann. Gegebenenfalls wird der Innenschaft 20 zusätzlich um seine Achse 22 gedreht, um die Ausrichtung in eine beliebige Raumrichtung zu bewirken. Der elastische erste Abschnitt 38 der Unterlage verursacht eine Rückstellkraft und ein Zurückschwenken des NMR-Sensors 30 in die in Fig. 26 gezeigte Ausgangslage, wenn die Zugkraft F auf das Zugseil 37 verringert wird.

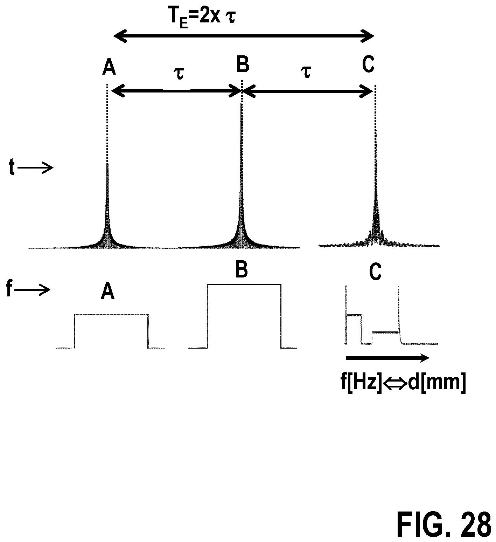

Aufgrund des vergleichsweise starken und nichtlinearen statischen Magnetfeldgradienten eines als kugelförmiger Festkörpermagnet ausgebildeten ersten Sensorelements 34 werden die angeregten Spins in kurzer Zeit dephasieren. sodass eine Aufnahme der Signalantwort entsprechend erschwert ist. Diesem Umstand lässt sich mittels Spinecho-Verfahren entgegenwirken, bei dem z. B. nach einem 90°-Anregungspuls ein weiterer Puls gesendet wird, der die Spins der Protonen um 180° umkehrt (vergleiche Fig. 28). Die Dauer jedes Magnetfeldpulses beträgt üblicherweise einige Millisekunden. Jeder Magnetfeldpuls ist ein Breitbandpuls über einen Frequenzbereich von beispielsweise 1 kHz bis 20 MHz. Die Anregung mit den Pulsen A und B sowie das NMR-Signal C aus dem Gewebe sind in Fig. 28 oben in der Zeitdomäne und unten in der Frequenzdomäne dargestellt.

Die vorliegende Erfindung nutzt die bekannte NMR-Technologie um auf einfache und kostengünstige Weise eine Aussage über den Fortschritt einer Behandlung bzw. die Größe einer Läsion, insbesondere ihre Tiefe in dem Gewebe zu treffen. Mit der erfindungsgemäßen Lösung kann durch die Gestaltung des NMR-Sensors 30 die NMR-Anregung auf einen Anregungskegel 32 mit einem kleinen Öffnungswinkel beschränkt werden. Über die Magnetfeldparameter kann die Tiefe des Betrachtungsfeldes beeinflusst werden.

Bezugszeichenliste

| | |
|---|---|
| 1 | Griff des Katheters |
| 2 | Signalleitung |
| 3 | Spülleitung |
| 4 | Steuermechanismus |
| 5 | Elektrode |
| 20 | Innenschaft |
| 22 | Achse (Längsachse) des Innenschafts |
| 25 | Schaftspitze |
| 26 | Kreuzschlitz |
| 27 | spiralförmiger Schlitz |
| 29 | Helixantenne |
| 30 | NMR-Sensor |
| 32 | Anregungskegel |
| 34 | erstes Sensorelement |
| 34a | Arm des Hufeisenmagnets |
| 35 | zweites Sensorelement |
| 35a | Spulenkern |
| 37 | Zugseil |
| 38 | erster Abschnitt der Unterlage |

| | |
|---|---|
| 39 | zweiter Abschnitt der Unterlage |
| 40 | Datenverarbeitungseinrichtung |
| 50 | Herzmuskelgewebe |
| A,B | Anregungspuls |
| BR | Breite |
| C | NMR-Signal |
| F | Kraft |
| H | Höhe |
| P1 | Pfeil 1 |
| P2 | Pfeil 2 |
| f | Darstellung in Frequenzdomäne |
| t | Darstellung in Zeitdomäne |

**Patentansprüche**

1. Vorrichtung zur Bestimmung einer lokalen Eigenschaft eines biologischen Gewebes (50) mit

   - einem flexiblen Schaft (20),
   - einer Datenverarbeitungseinrichtung (40) und
   - einem NMR-Sensor (30), der an dem distalen Ende des Schafts (20) angeordnet und mit der Datenverarbeitungseinrichtung (40) verbunden ist, wobei der NMR-Sensor (30) ein erstes Sensorelement (34) zur Erzeugung eines statischen Magnetfelds und ein zweites Sensorelement (35) zur Erzeugung eines magnetischen Wechselfelds aufweist,

   wobei das distale Ende des Schafts (20) benachbart zu der zu vermessenden Stelle des Gewebes (50) anordenbar ist, wobei die Datenverarbeitungseinrichtung (40) zur Bestimmung einer lokalen Eigenschaft des Gewebes (50) an dieser Stelle basierend auf einem zur Datenverarbeitungseinrichtung (40) übertragenen Signal des NMR-Sensors (30) eingerichtet ist.

2. Vorrichtung nach Anspruch 1, **dadurch gekennzeichnet, dass** das erste Sensorelement (34) als Permanentmagnet, der beispielsweise kugelförmig oder quaderförmig gestaltet ist, oder als Spule ausgebildet ist.

3. Vorrichtung nach einem der vorhergehenden Ansprüche, **dadurch gekennzeichnet, dass** das zweite Sensorelement (35) als Spule ausgebildet ist.

4. Vorrichtung nach einem der vorhergehenden Ansprüche, **dadurch gekennzeichnet, dass** eine an dem distalen Ende des Schafts (20) angeordnete Schaftspitze (25) mindestens eine schlitzförmige Ausnehmung (26, 27) aufweist oder als Helixantenne (29) gestaltet ist.

5. Vorrichtung nach einem der vorhergehenden Ansprüche, **dadurch gekennzeichnet, dass** der NMR-Sensor (30) mittels mindestens eines an dem NMR-Sensor (30) befestigten Zugseils (37) gegenüber dem Schaft (20) schwenkbar und/oder drehbar ist.

6. Vorrichtung nach einem der vorhergehenden Ansprüche, **dadurch gekennzeichnet, dass** der NMR-Sensor (30) auf einer Unterlage gelagert ist, welche einen ersten Abschnitt (38) mit einer höheren Elastizität und einen zweiten Abschnitt (39) mit einer verglichen mit dem ersten Abschnitt (38) geringeren Elastizität aufweist, wobei der erste Abschnitt bei einem Verschwenken des NMR-Sensors (30) gegenüber dem Schaft (20) eine Rückstellkraft bewirkt.

7. Vorrichtung nach einem der vorhergehenden Ansprüche, **dadurch gekennzeichnet, dass** der NMR-Sensor (30) zur Anregung mittels magnetischer Wechselfeldpulse eingerichtet ist, wobei nach einem 90° Anregungspuls ein weiter Puls gesendet wird, der die Spins der Protonen des Gewebes um 180° dreht.

8. Katheter aufweisend eine Vorrichtung nach einem der vorhergehenden Ansprüche.

9. Verfahren zur Bestimmung einer lokalen Eigenschaft eines biologischen Gewebes (50) bei dem nach Anregung durch einen benachbart zu der zu vermessenden Stelle des Gewebes (50) an dem distalen Ende eines flexiblen Schafts (20) angeordneten NMR-Sensor (30) ein NMR-Antwortsignal des Gewebes (50) erzeugt und basierend auf diesem NMR-Antwortsignal die lokale Gewebeeigenschaft bestimmt wird.

10. Verfahren nach Anspruch 9, **dadurch gekennzeichnet, dass** vor der Erzeugung des NMR-Signals die Achse eines Anregungskegels (32) des NMR-Sensors (30) im Wesentlichen senkrecht zur Gewebeoberfläche ausgerichtet wird.

11. Verfahren nach Anspruch 10, **dadurch gekennzeichnet, dass** die Ausrichtung des NMR-Sensors (30) erfolgt

- durch Betätigen mindestens eines an dem NMR-Sensor (30) befestigten Zugseils (37), so dass ein Schwenken und/oder Drehen des NMR-Sensors (30) bewirkt wird, und/oder
- durch Drehen des Schafts (20).

12. Verfahren nach einem der Ansprüche 9 bis 11, **dadurch gekennzeichnet, dass** das distale Ende des Schafts (20) derart in Richtung der Längsachse des Schafts (20) verschoben wird, dass das distale Ende des Schafts (20) an der Oberfläche des zu vermessenden Gewebes (50) anliegt.

13. Verfahren nach einem der Ansprüche 9 bis 12, **dadurch gekennzeichnet, dass** intermittierend zwischen der Bestimmung der lokalen Gewebeeigenschaft basierend auf dem NMR-Signal ein an dem distalen Ende des Schafts (20) angeordnete Schaftspitze (25) mit einem Strom versorgt oder an der Schaftspitze (25) eine Spannung angelegt wird.

14. Computerprogrammprodukt zur Bestimmung einer lokalen Eigenschaft eines biologischen Gewebes mit Programmcodemitteln zur Ausführung eines Computerprogramms nach dessen Implementierung in einer Datenverarbeitungseinrichtung (40), **dadurch gekennzeichnet, dass** die Programmcodemittel dazu vorgesehen sind, nach der Implementierung in der Datenverarbeitungseinrichtung (40) das Verfahren nach einem der Ansprüche 9 bis 13 auszuführen.

FIG. 1

**40**

**20**

**25**

**30**

**32**

# FIG. 2

**35**

**34**

w

6,5

**22**

**20**

# FIG. 3

FIG. 4

FIG. 5

FIG. 6

FIG. 7

25

FIG. 8

25

26

FIG. 9

25

27

FIG. 10

25

27

FIG. 11

34

20

30

34

35

**FIG. 12**          **FIG. 13**

25

**FIG. 14**

25

29

**FIG. 16**

**FIG. 15**

P1

P2

37

37

37

37

**FIG. 17**

50

32

20

**FIG. 18**

32

50

20

**FIG. 19**

32

20

**FIG. 20**

34

P1

34

20

20

37

37

37

37

$\vec{F}$

## Fig. 21

## Fig. 22

35  35a  34a

34a

N  S

34

H

BR

FIG. 23

FIG. 24

FIG. 25

FIG. 26

FIG. 27

FIG. 28

Europäisches Patentamt

European Patent Office

Office européen des brevets

**EUROPÄISCHER RECHERCHENBERICHT**

Nummer der Anmeldung

EP 17 20 6949

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 704 594 B1 (BLANK AHARON [IL] ET AL) 9. März 2004 (2004-03-09) | 1-3,5, 7-11,14 | INV. G01R33/28 G01R33/38 |
| Y | * Spalte 3, Zeile 32 - Spalte 6, Zeile 19; Abbildungen 1,2,7, 12 * <br> * Spalte 9, Zeile 37 - Spalte 10, Zeile 62 * <br> * Spalte 17, Zeile 34 - Zeile 39; Ansprüche 1-8, 13,14 * <br> * Spalte 12, Zeile 17 - Zeile 53 * <br> * Spalte 14, Zeile 14 - Zeile 17 * <br> ----- | 5,6 | |
| X | US 2005/021019 A1 (HASHIMSHONY DAN [IL] ET AL) 27. Januar 2005 (2005-01-27) | 1-4,8-14 | |
| Y | * Absätze [0004], [0030], [0041], [0042], [0068] - [0101], [0148] - [0151]; Abbildungen 1-5, 10-13 * <br> ----- | 5,6 | |
| X | US 2004/158144 A1 (KEREN HANAN [IL] ET AL) 12. August 2004 (2004-08-12) | 1-3,5, 7-9,14 | |
| Y | * Absätze [0006], [0007], [0023] - [0027], [0040] - [0042], [0071] - [0081]; Anspruch 1; Abbildungen 2,5-17 * <br> ----- | 5,6 | RECHERCHIERTE SACHGEBIETE (IPC) |
| Y | US 8 260 399 B2 (KARMARKAR PARAG V [US]; ATALAR ERGIN [US]; UNIV JOHNS HOPKINS [US]) 4. September 2012 (2012-09-04) * Spalte 2, Zeile 62 - Spalte 3, Zeile 35 * <br> * Spalte 5, Zeile 56 - Spalte 6, Zeile 28 * <br> * Spalte 9, Zeile 4 - Zeile 18; Anspruch 1; Abbildungen 3,6 * <br> ----- | 5,6 | G01R A61B |

Der vorliegende Recherchenbericht wurde für alle Patentansprüche erstellt

| Recherchenort | Abschlußdatum der Recherche | Prüfer |
|---|---|---|
| München | 25. Juni 2018 | Faber-Jurk, Sonja |

EPO FORM 1503 03.82 (P04C03)

**EP 3 499 258 A1**

**ANHANG ZUM EUROPÄISCHEN RECHERCHENBERICHT**
**ÜBER DIE EUROPÄISCHE PATENTANMELDUNG NR.**

EP 17 20 6949

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-06-2018

| Im Recherchenbericht angeführtes Patentdokument | Datum der Veröffentlichung | Mitglied(er) der Patentfamilie | Datum der Veröffentlichung |
|---|---|---|---|
| US 6704594 B1 | 09-03-2004 | AU 1084702 A<br>EP 1340093 A1<br>JP 3872431 B2<br>JP 2004522472 A<br>JP 2007014794 A<br>US 6704594 B1<br>WO 0239132 A1 | 21-05-2002<br>03-09-2003<br>24-01-2007<br>29-07-2004<br>25-01-2007<br>09-03-2004<br>16-05-2002 |
| US 2005021019 A1 | 27-01-2005 | CA 2533161 A1<br>CN 1852678 A<br>EP 1653852 A2<br>JP 4777886 B2<br>JP 2007507245 A<br>US 2005021019 A1<br>WO 2005009200 A2 | 03-02-2005<br>25-10-2006<br>10-05-2006<br>21-09-2011<br>29-03-2007<br>27-01-2005<br>03-02-2005 |
| US 2004158144 A1 | 12-08-2004 | KEINE | |
| US 8260399 B2 | 04-09-2012 | US 2004220470 A1<br>US 2010198049 A1<br>WO 2004068947 A2 | 04-11-2004<br>05-08-2010<br>19-08-2004 |

EPO FORM P0461

Für nähere Einzelheiten zu diesem Anhang : siehe Amtsblatt des Europäischen Patentamts, Nr.12/82

26

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10309245 A1
- US 2014324085 A1
- US 2015209551 A1
- US 2015196202 A1
- WO 2014163974 A2